# EUROPÄISCHE PATENTANMELDUNG

(11) **EP 0 842 941 A1**
(43) Veröffentlichungstag der Anmeldung: **20.05.1998**
(21) Anmeldenummer: 96118410.8
(22) Anmeldetag: 16.11.1996
(51) Int. Cl.: C07F 9/59, A61K 31/675, C07F 9/572, C07F 9/553

(54) **Neue Phosphonate, Verfahren zu ihrer Herstellung und Arzneimittel**

(71) Anmelder: BOEHRINGER MANNHEIM GMBH, 68298 Mannheim (DE)
(72) Erfinder: Kucznierz, Ralf, Dr., Dipl-Chemiker, 68167 Mannheim (DE); Leinert, Herbert, Dr., Dipl.-Chemiker, 64646 Heppenheim (DE); Von der Saal, Wolfgang, Dr., Dipl.-Chemiker, 69469 Weinheim (DE); Neidlein, R., Prof. Dr., Pharm.-Chem., 69120 Heidelberg (DE); Kehr, Christiane, Dipl.-Chemikerin, 69120 Heidelberg (DE)

(57) **Zusammenfassung**

Verbindungen der Formel I 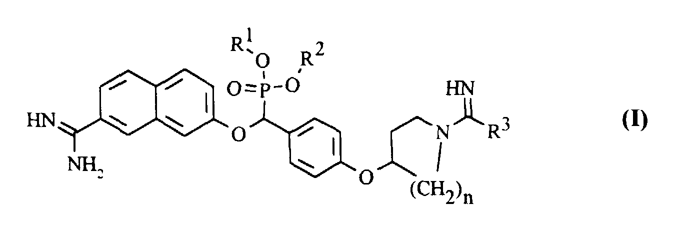 in der
- R¹, R²: gleich oder verschieden sind und ein Wasserstoffatom, eine Alkylgruppe, eine Cycloalkylgruppe, eine Hydroxyalkylgruppe, eine Alkenylgruppe, eine Alkinylgruppe oder eine Aralkylgruppe bedeuten oder R¹ und R² zusammen einen Alkylenrest bedeuten, der mit den gebundenenen Sauerstoffatomen und dem die Sauerstoffatome tragenden Phosphoratom einen gesättigten 5- bis 8-gliedrigen Ring bildet;
- R³: eine gegebenenfalls substituierte Aminogruppe, eine Alkylgruppe, einen Cycloalkylrest oder einen gegebenenfalls substituierten Arylrest bedeutet;
- n: eine ganze Zahl zwischen 1 und 4 bedeutet,
sowie Hydrate, Solvate und physiologisch verträgliche Salze davon, deren optisch aktiven Formen, Verfahren zu ihrer Herstellung sowie Arzneimittel mit Faktor Xa-inhibierenden Eigenschaften, die diese Verbindungen enthalten.

## Beschreibung

Die Erfindung betrifft neue Phosphonate der allgemeinen Formel I 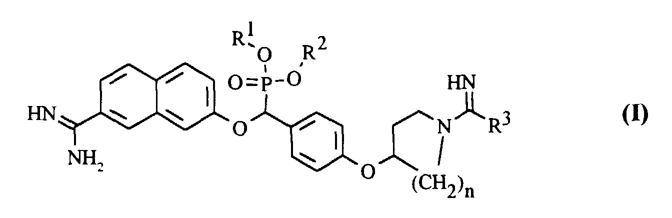 in der
- R¹, R²: gleich oder verschieden sind und ein Wasserstoffatom, eine Alkylgruppe, eine Cycloalkylgruppe, eine Hydroxyalkylgruppe, eine AIkenylgruppe, eine Alkinylgruppe oder eine Aralkylgruppe bedeuten oder R¹ und R² zusammen einen Alkylenrest bedeuten, der mit den gebundenenen Sauerstoffatomen und dem die Sauerstoffatome tragenden Phosphoratom einen gesättigten 5- bis 8-gliedrigen Ring bildet;
- R³: eine gegebenenfalls substituierte Aminogruppe, eine Alkylgruppe, einen Cycloalkylrest oder einen gegebenenfalls substituierten Arylrest bedeutet;
- n: eine ganze Zahl zwischen 1 und 4 bedeutet,
sowie Hydrate, Solvate und physiologisch verträgliche Salze davon. Gegenstand der Erfindung sind auch die optisch aktiven Formen, die Racemate und die Diastereomerengemische dieser Verbindungen.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln.

Die Phosphonate der allgemeinen Formel I, ihre Solvate und ihre Salze greifen durch reversible Inhibierung von Faktor Xa in den Prozeß der Blutgerinnung ein und verhindem somit die Entstehung von Gerinnungsthromben. Sie können deshalb bei der Bekämpfung und Verhütung von Krankheiten, wie Thrombose, Apoplexie, Herzinfarkt, Entzündungen und Arteriosklerose, verwendet werden.

Faktor Xa ist eine Serinprotease des Gerinnungssystems, die die proteolytische Umwandlung von Prothrombin in Thrombin katalysiert. Thrombin, als letztes Enzym der Gerinnungskaskade, spaltet einerseits Fibrinogen zu Fibrin, das nach Quervernetzung mittels Faktor XIIIa zu einem unlöslichen Gel wird und die Matrix für einen Thrombus bildet, andererseits aktiviert es durch Proteolyse seines Rezeptors auf den Blutplättchen die Plättchenaggregation und trägt auf diesem Weg ebenfalls zur Thrombusbildung bei. Bei der Verletzung eines Blutgefäßes sind diese Prozesse notwendig, um eine Blutung zu stoppen. Unter normalen Umständen sind keine meßbaren Thrombin-Konzentrationen im Blutplasma vorhanden. Ein Ansteigen der Thrombinkonzentration kann zur Ausbildung von Thromben und damit zu thromboembolischen Krankheiten führen, die vor allem in den Industriestaaten sehr häufig auftreten. Durch Hemmung von Faktor Xa kann die Entstehung von Thrombin verhindert werden.

Kürzlich wurde berichtet, daß Amidinoarylpropansäure-Derivate wie (+)-(2S)-2-[4-[[(3S)-1-Acetimidoyl-3-pyrrolidinyl]oxy]phenyl]-3-(7-amidino-2-naphthyl]propansäure-hydrochlorid-pentahydrat (DX-9065a; Formel IIa) Faktor Xa inhibieren (*J. Med. Chem*. **1994**, *37*, 1200-1207; *Thrombosis and Haemostasis* **1994**, *71*, 314-319; EP-0-540-051-A-1). Weitere bekannte Faktor-Xa-Inhibitoren sind 1,2-Bis-(5-amidino-2-benzofuranyl)-ethan (DABE, Formel IIb, *Thrombosis Research* **1980**, *19*, 339-349) oder auch Phenyl-amino-methyl-naphthamidine der allgemeinen Formel IIc (WO96/16940).

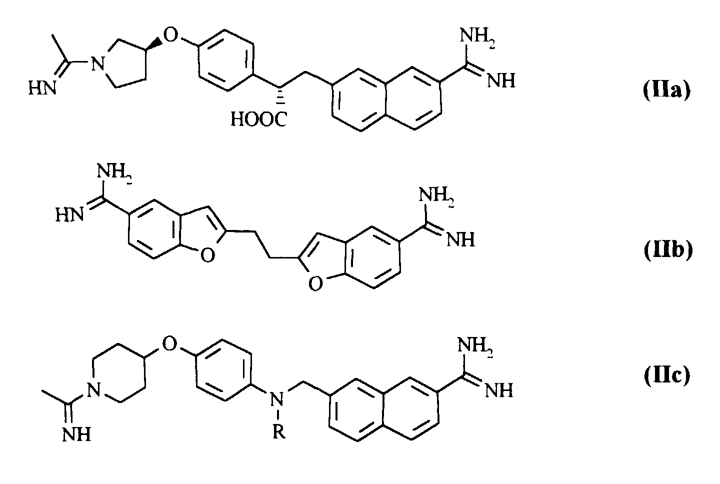

Die erfindungsgemäßen, neuen Phosphonate der allgemeinen Formel I sowie Hydrate, Solvate und physiologisch verträgliche Salze davon sind potente und selektive Faktor Xa-Inhibitoren.

In der allgemeinen Formel I können die Substituenten R¹ und R² gleich oder verschiedenartig sein.

Bedeutet R¹, R² in der allgemeinen Formel I eine Alkylgruppe, so kann diese geradkettig oder verzweigt sein und 1 bis 6 Kohlenstoffatome enthalten. Bevorzugt sind die Methyl-, Ethyl-, *n*-Propyl-, *i*-Propyl-, *n*-Butyl-, *i*-Butyl-, *t*-Butyl-, Pentyl- und die Hexylgruppe.

Bedeutet R¹, R² in der allgemeinen Formel I eine Cycloalkylgruppe, so kann diese substituiert oder unsubstituiert sein und 3 bis 8 Kohlenstoffatome enthalten. Bevorzugt sind die Cyclopropyl-, Cyclopentyl-, Cyclohexyl- und die Cyclooctylgruppe.

Bedeutet R¹, R² in der allgemeinen Formel I eine Hydroxyalkylgruppe, so kann diese geradkettig oder verzweigt sein und 1 bis 6 Kohlenstoffatome enthalten. Bevorzugt sind die Hydroxymethyl-, Hydroxyethyl-, Hydroxypropyl-, Hydroxybutyl-, Hydroxypentyl- und die Hydroxyhexylgruppe.

Bedeutet R¹, R² in der allgemeinen Formel I eine Alkenylgruppe, so kann diese geradkettig oder verzweigt sein und 2 bis 6 Kohlenstoffatome enthalten. Bevorzugt sind die Vinyl-, 1-Propenyl-, 2-Propenyl-, 2-Methyl-2-propenyl-, 1-Butenyl-, 1-Pentenyl- und die 1-Hexenylgruppe.

Bedeutet R¹, R² in der allgemeinen Formel I eine Alkinylgruppe, so kann diese geradkettig oder verzweigt sein und 2 bis 6 Kohlenstoffatome enthalten. Bevorzugt sind die Ethinyl- und die Propargylgruppe.

Bedeutet R¹, R² in der allgemeinen Formel I eine Aralkylgruppe, so enthält diese eine mit einer geradkettigen oder verzweigten C₁-C₆-Alkylkette verknüpfte Phenylgruppe, eine mit einer geradkettigen oder verzweigten C₁-C₆-Alkylkette verknüpfte Naphthylgruppe oder eine mit einer geradkettigen oder verzweigten C₁-C₆-Alkylkette verknüpfte Biphenylgruppe. Bevorzugt sind dabei die Berzylgruppe, die p-Phenylbenzylgruppe und die Naphthylmethylgruppe.

Bedeuten R¹ und R² zusammnen in der allgemeinen Formel I eine Alkylengruppe, so kann diese geradkettig oder verzweigt sein und 2 bis 6 Kohlenstoffatome enthalten. Bevorzugt sind die Ethylen-, Propylen- und die 2,3-Dimethyl-2,3-butandiylgruppe.

Bedeutet R³ in der allgemeinen Formel I eine Aminogruppe, so kann diese unsubstituiert oder auch substituiert sein und zwar mit einer oder zwei C₁-C₆-Alkylgruppen, vorzugsweise Methyl oder Ethyl, mit einer oder zwei C₃-C₈-Cycloalkylgruppen, vorzugsweise Cyclopropyl, Cyclopentyl, Cyclohexyl oder Cyclooctyl, mit einer oder zwei C₁-C₆-Hydroxyalkylgruppen, vorzugsweise Hydroxyethyl oder Hydroxypropyl, mit einer oder zwei C₃-C₆-Alkenylgruppen, vorzugsweise Allyl, mit einer oder zwei C₃-C₆-Alkinyl-gruppen, vorzugsweise Propargyl oder mit einer oder zwei Alalkylgruppen, vorzugsweise Benzyl. Die Spezifizierung (C₁-C₆) steht hier jeweils für eine geradkettige oder verzweigte Alkylkette mit 1 bis 6 Kohlenstoffatomen, (C₃-C₈) bezeichnet eine verzweigte oder unverzweigte Cycloalkylgruppe mit 3 bis 8 Kohlenstoffatomen und (C₃-C₆) kennzeichnet wahlweise eine verzweigte oder unverzweigte Alkenyl- oder Alkinylgruppierung mit 3 bis 6 Kohlenstoffatomen.

Bedeutet R³ in der allgemeinen Formel I eine Alkylgruppe, so kann diese geradkettig oder verzweigt sein und 1 bis 6 Kohlenstoffatome enthalten. Bevorzugt sind die Methyl-, Ethyl-, *n*-Propyl-, *i*-Propyl-, *n*-Butyl-, *i*-Butyl-, *t*-Butyl-, Pentyl- und die Hexylgruppe.

Bedeutet R³ in der allgemeinen Formel I eine Cycloalkylgruppe, so kann diese verzweigt oder unverzweigt sein und 3 bis 8 Kohlenstoffatome enthalten. Bevorzugt sind die Cyclopropyl-, Cyclopentyl-, Cyclohexyl- und die Cyclooctylgruppe.

Bedeutet R³ in der allgemeinen Formel I einen Arylrest, so versteht man darunter die Phenyl-, eine Biphenyl- oder eine Naphthylgruppe. Der Arylrest kann unsubstituiert sein oder gegebenenfalls einen oder mehrere C₁-C₆-Alkyl-, vorzugsweise Methyl, C₁-C₆-Alkyloxy-, vorzugsweise Methoxy, oder Halogensubstituenten tragen. Die Spezifizierung (C₁-C₆) steht hier jeweils für eine geradkettige oder verzweigte Alkylkette mit 1 bis 6 Kohlenstoffatomen. Halogene als Substituenten des Arylrestes können Fluor-, Chlor-, Brom- und Iodatome, bevorzugt jedoch Chlor- oder Bromatome sein.

Die Zahl n bedeutet eine ganze Zahl zwischen 1 und 4.

Bevorzugt sind Verbindungen der allgemeinen Formel I, in denen
- R¹, R²: gleich oder verschieden sind und ein Wasserstoffatom, eine Methylgruppe, eine Ethylgruppe, eine Propylgruppe, eine Allylgruppe, eine Propargylgruppe oder eine Benzylgruppe bedeuten, oder R¹ und R² zusammen eine Ethylengruppe oder eine Propylengruppe bedeuten;
- R³: eine Aminogruppe, eine N-Methyl-aminogruppe, eine N-Benzyl-aminogruppe, eine N-Allyl-aminogruppe, eine N,N-Dimethylaminogruppe, eine Methylgruppe, eine Ethylgruppe, eine Cyclopropylgruppe oder einen 4-Methoxyphenylrest bedeutet und
- n: 1 oder 2 sein kann.

Besonders bevorzugt sind Verbindungen der allgemeinen Formel I, in denen
- R¹ und R²: gleich sind und eine Ethylgruppe bedeuten;
- R³: eine Methylgruppe bedeutet und
- n: die Zahl 2 bedeutet.

Unter den physiologisch verträglichen Salzen der allgemeinen Formel I versteht man beispielsweise Formiate, Acetate, Caproate, Oleate, Lactate oder Salze von Carbonsäuren mit bis zu 18 Kohlenstoffatomen oder Salze von Dicarbonsäuren und Tricarbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure, Iodwasserstoffsäure, Schwefelsäure, Phosphorsäure. Die Verbindungen der Formel I mit einer oder zwei freien Säuregruppen am Phosphonatfragment können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-, Calcium- oder Tetramethylammoniumsalz.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten.

Gegenstand der Erfindung sind auch die optisch aktiven Formen. die Racemate und die Diastereomerengemische von Verbindungen der allgemeinen Formel I.

Zur Herstellung von Arzneimitteln werden die Substanzen der allgemeinen Formel I mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, z.B. in Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethylenoxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewunschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch einmal pro Tag 1-2 Tabletten mit 20-700 mg Wirkstoff gegeben werden müssen. Der Wirkstoff kann auch durch Injektion 1-8 mal pro Tag oder durch Dauerinfusion gegeben werden, wobei 50-2000 mg pro Tag normalerweise ausreichen.

Die Herstellung von Verbindungen der allgemeinen Formel I erfolgt nach an sich bekannten Methoden.

Die Verbindungen der allgemeinen Formel I werden hergestellt, indem man beispielsweise eine Verbindung der allgemeinen Formel III 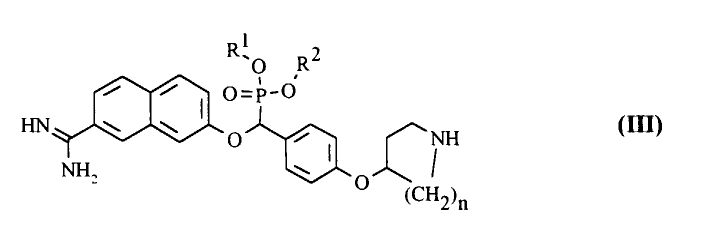 in der R¹, R² und n die oben angegebenen Bedeutungen besitzen, mit einem Guanylierungsreagenz wie beispielsweise 1H-Pyrazol-1-carboxamidin oder S-Methylisothioharnstoff in einem inerten Lösungsmittel wie z. B. Dimethylformamid, Dioxan, Dimethylsulfoxid oder Toluol bei Temperaturen zwischen 0°C und Siedepunkt des Lösungsmittels, vorzugsweise bei 0 bis 30°C in Gegenwart einer Hilfsbase wie z. B. Triethylamin, N-Methylmorpholin, Pyridin oder Ethyldiisopropylamin zur Reaktion bringt.

Verbindungen der allgemeinen Formel I können auch durch Umsetzung von Verbindungen der allgemeinen Formel III, in der R¹, R² und n die oben angegebenen Bedeutungen besitzen, mit entsprechend substituierten Guanylierungsreagensien in einem inerten Lösungsmittel wie z. B. Dimethylformamid, Dioxan, Dimethylsulfoxid oder Toluol bei Temperaturen zwischen 0°C und Siedepunkt des Lösungsmittels, vorzugsweise bei 0 bis 30°C in Gegenwart einer Hilfsbase wie z. B. Triethylamin, N-Methylmorpholin, Pyridin oder Ethyldiisopropylamin hergestellt werden.

Verbindungen der allgemeinen Formel I können auch durch Umsetzung von Verbindungen der allgemeinen Formel III, in der R¹, R² und n die oben angegebenen Bedeutungen besitzen, mit aliphatischen oder aromatischen Imidsäureester-Hydrochloriden in einem inerten Lösungsmittel wie Tetrahydrofuran, Diethylether, Ethanol, Dimethylformamid oder Dioxan in Gegenwart einer Hilfsbase wie Triethylamin, Ethyldiisopropylamin oder N-Methylmorpholin hergestellt werden.

Die Verbindungen der allgemeinen Formel III werden hergestellt, indem man eine Verbindung der allgemeinen Formel IV 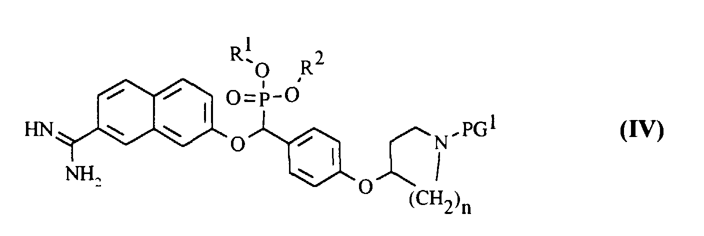 in der in der R¹, R² und n die oben angegebenen Bedeutungen besitzen und PG¹ eine Schutzgruppe wie z. B. die Benzyloxycarbonylgruppe, die t-Butyloxycarbonylgruppe oder die Allyloxycarbonylgruppe bedeutet, mit einem die Schutzgruppe abspaltenden Reagenz zur Reaktion bringt. Die Schutzgruppenabspaltung erfolgt nach allgemein üblichen Methoden (siehe z. B.: T. W. Green, P. G. M. Wuts, "Protective Groups in Organic Synthesis", 2nd ed., John Wiley and Sons Inc. 1991) durch saure Reagentien wie z.B. Bromwasserstoff in Eisessig oder Trifluoressigsäure oder etherische HCl-Lösung oder hydrogenolytisch oder durch palladium- oder rhodiumkatalysierte Spaltung.

Die Verbindungen der allgemeinen Formel IV werden hergestellt, in dem man eine Verbindung der allgemeinen Formel V 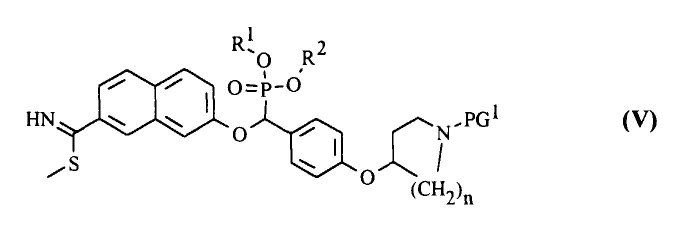 in der R¹, R², n und PG¹ die oben angegebenen Bedeutungen besitzen, mit Ammoniak oder Ammoniumsalzen wie z. B. Ammoniumacetat, Ammoniumchlorid oder Ammoniumoxalat in einem inerten Lösungsmittel wie Methanol, Ethanol oder Isopropanol bei Temperaturen zwischen 0°C und dem Siedepunkt des Lösungsmittels, vorzugsweise zwischen Raumtemperatur und 65°C, umsetzt.

Verbindungen der allgemeinen Formel V stellt man her durch Umsetzung einer Verbindung der allgemeinen Formel VI 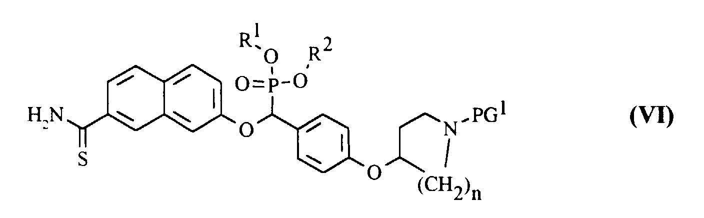 in der R¹, R², n und PG¹ die oben angegebenen Bedeutungen besitzen, mit Methylienungsreagentien wie Methyliodid oder Dimethylsulfat in einem inerten Lösungsmittel wie Methanol, Ethanol, Aceton oder Dioxan bei Temperaturen zwischen 0°C und dem Siedepunkt des Lösungsmittels, vorzugsweise zwischen Raumtemperatur und 55°C.

Verbindungen der allgemeinen Formel VI erhält man durch Behandlung einer Verbindung der allgemeinen Formel VII 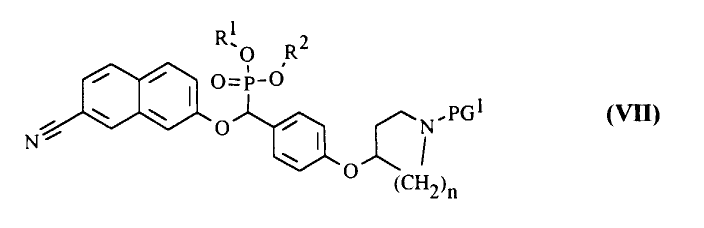 in der R¹, R², n und PG¹ die oben angegebenen Bedeutungen besitzen, mit Schwefelwasserstoff in einem inerten Lösungsmittel wie Pyridin, methanolischer Ammoniaklösung, Ethanol, Chloroform oder Dimethylformamid bei Temperaturen zwischen 0°C und dem Siedepunkt des Lösungsmittels, vorzugsweise zwischen 0°C und Raumtemperatur, gegebenenfalls in Gegenwart einer Hilfsbase wie Diethylamin, Triethylamin, Ethyldiisopropylamin oder N-Methylmorpholin.

Die Verbindungen der allgemeinen Formel VII stellt man her, indem man 7-Hydroxy-naphthalin-2-carbonitril mit einer Verbindung der allgemeinen Formel VIII 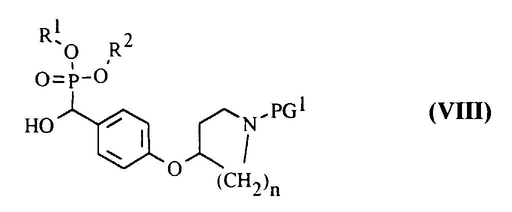 in der R¹, R², n und PG¹ die oben angegebenen Bedeutungen haben in einem inerten Lösungsmittel wie Dioxan, Tetrahydroturan oder Toluol in Gegenwart von Diethylazodicarboxylat und Triphenylphosphin, Trimethyl- oder Triethylphosphit bei Temperaturen zwischen 0 und 50°C, vorzugsweise bei Raumtemperatur, kondensiert.

7-Hydroxy-naphthalin-2-carbonitril ist literaturbekannt (siehe z. B.: L. M. Tolbert, J. E. Haubrich, *J. Am. Chem. Soc.* **1994**, *116*, 10593-10600) und kann nach den dort oder an anderer Stelle beschriebenen Verfahren (z. B.: S. A. Jacobs, R. G. Harvey, *J. Org. Chem*. **1983**, *48*, 5134-5135; V. N. Kopranenkov, E. A. Makarova, E. A. Luk'yanets, *Zh*. *Org*. *Khim*. **1981**, 358-361; B. Basu, D. Mukherjee, *J. Chem*. *Soc*. *Chem*. *Commun*. **1984**, 105-106; B. Basu, D. Mukherjee, *Tetrahedron Lett*. **1984**, 4445-4446; S. K. Maity, D. Mukherjee, *Tetrahedron Lett*. **1983,** 5919-5920) hergestellt werden.

Die Verbindungen der allgemeinen Formel VIII werden hergestellt, indem man eine Verbindung der allgemeinen Formel IX 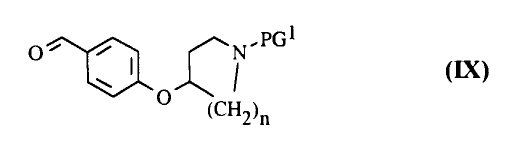 in der n und PG¹ die oben angegebenen Bedeutungen haben, mit einer Verbindung der allgemeinen Formel X 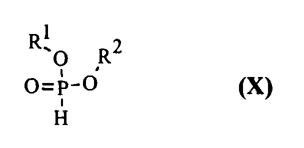 in der R¹ und R² die oben angegebenen Bedeutungen besitzen, in Gegenwart einer Hilfsbase wie Triethylamin, Diisopropylethylamin, N-Methylmorpholin, Pyridin, Natriummethylat oder Natriumethylat bei Temperaturen zwischen 0°C und 80°C, vorzugsweise bei 60°C, umsetzt.

Verbindungen der allgemeinen Formel X, in der R¹ und R² die oben angegebenen Bedeutungen besitzen, sind entweder käuflich oder literaturbekannt oder können aus käuflichen oder literaturbekannten Vorstufen nach Standardmethoden hergestellt werden.

Die Verbindungen der allgemeinen Formel IX stellt man her, in dem man eine Verbindung der allgemeinen Formel XI, 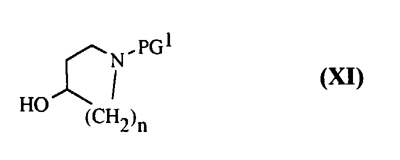 in der n und PG¹ die oben angegebenen Bedeutungen haben, in einem inerten Lösungsmittel wie Dioxan, Tetrahydrofuran oder Toluol in Gegenwart von Diethylazodicarboxylat und Triphenylphosphin, Trimethyl- oder Triethylphosphit bei Temperaturen zwischen 0 und 50°C, vorzugsweise bei Raumtemperatur, mit 4-Hydroxyberzaldehyd kondensiert.

Verbindungen der allgemeinen Formel XI, in der n und PG¹ die oben angegebenen Bedeutungen haben, sind entweder käuflich oder literaturbekannt oder können nach literaturbekannten Verfahren hergestellt werden (siehe z. B.: K. L. Bhat, D. M. Flanagan, M. M. Jouillé, *Synth*. *Commun*. **1985**, *15*, 587-598; P. G. Houghton, G. R. Humphrey, D. J. Kennedy, D. C. Roberts, S. H. Wright, *J. Chem. Soc. Perkin Trans.1* **1993**, *13*, 1421-1424; T. W. Green, P. G. M. Wuts, "Protective Groups in Organic Synthesis", 2nd ed., John Wiley and Sons Inc. 1991).

Gewisse Verbindungen der allgemeinen Formel I lassen sich nachträglich in andere Verbindungen der allgemeinen Formel I umwandeln.

Dies betrifft Verbindungen der allgemeinen Formel I, in der n und PG¹ die oben angegebenen Bedeutungen haben und R¹ und R² gleich oder verschieden sind und eine Alkylgruppe, eine Cycloalkylgruppe, eine Alkenylgruppe, eine Alkinylgruppe oder eine Aralkylgruppe bedeuten. Durch partielle Hydrolyse, z. B. mit wäßriger Kalilauge, mit wäßrigem Pyridin oder durch Behandlung mit Natriumiodid in Aceton bei Temperaturen zwischen 0°C und dem Siedepunkt des Lösungsmittels, vorzugsweise bei Rückflußtemperatur, können diese Verbindungen in Verbindungen der allgemeinen Formel I mit einer freien Phosphonsäuregruppe umgewandelt werden.

Dies betrifft auch Verbindungen der allgemeinen Formel I, in der n und PG¹ die oben angegebenen Bedeutungen haben, R¹ eine Alkylgruppe oder eine Cycloalkylgruppe und R² die Benzylgruppe bedeutet. Durch katalytische Hydrierung in inerten Lösungsmitteln wie z. B. Methanol, Ethanol, Tetrahydrofuran oder Dioxan in Gegenwart eines Katalysators, vorzugsweise Palladium auf Kohle, wird die Berzylgruppe dabei durch ein Wasserstoffatom ersetzt. Die Entfernung der Berzylgruppe gelingt auch durch Umsetzung mit einer starken Säure wie Trifluoressigsäure in Gegenwart von Mesitylen, Anisol oder Thioanisol bei Temperaturen zwischen 0 und 50°C, vorzugsweise bei Raumtemperatur, oder durch Behandlung mit Lewissäuren wie BF₃-Etherat in einem inerten Lösungsmittel wie Toluol, Acetonitril, Diethylether oder Tetrahydrofuran bei Temperaturen zwischen 0°C und dem Siedepunkt des Lösungsmittels. vorzugsweise zwischen Raumtemperatur und dem Siedepunkt des Lösungsmittels.

Dies betrifft auch Verbindungen der allgemeinen Formel I, in der n und PG¹ die oben angegebenen Bedeutungen haben, R¹ eine Alkylgruppe oder eine Cycloalkylgruppe und R² die Allylgruppe bedeutet. Durch übergangsmetallkatalysierte Spaltung, beispielsweise in Gegenwart eines Rhodiumkatalysators wie Tris-triphenylphosphin-rhodiumchlorid oder eines Palladiumkatalysators wie Tetrakis-triphenylphosphin-palladium in einem inerten Lösungsmittel wie Tetrahydrofuran oder Dioxan, gegebenenfalls in Anwesenheit eines Nucleophils wie z. B. Malonsäurediethylester, Tributylzinnhydrid, 5,5-Dimethyl-cyclohexan-1,3-dion oder Piperidin bei Temperaturen zwischen 0°C und 50°C, vorzugsweise bei Raumtemperatur, wird die Allylgruppe dabei durch ein Wasserstoffatom ersetzt.

Dies betrifft auch Verbindungen der allgemeinen Formel I, in der n und PG¹ die oben angegebenen Bedeutungen haben und R¹ und R² gleich oder verschieden sind und eine Alkylgruppe, eine Cycloalkylgruppe, eine Hydroxyalkylgruppe, eine Alkenylgruppe, eine Alkinylgruppe oder eine Aralkylgruppe bedeuten. Durch Behandlung mit Trimethylsilyliodid oder Trimethylsilylbromid bei Temperaturen zwischen 0°C und dem Siedepunkt des Lösungsmittels, vorzugsweise zwischen Raumtemperatur und 50°C, in inerten Lösungsmitteln wie Diethylether, Tetrahydrofuran, Dimethylformamid, Dichlormethan oder Chloroform können diese Verbindungen in freie Phosphonsäuren der allgemeinen Formel I umgewandelt werden. Die vollständige Verseifung von Phosphonsäureestern der allgemeinen Formel I gelingt auch durch saure Hydrolyse in wäßrigem Medium, beispielsweise in verdünnter oder halbkonzentrierter Salzsäure bei Temperaturen zwischen Raumtemperatur und dem Siedepunkt des Reaktionsmediums, vorzugsweise bei Rückflußtemperatur.

Zu reinen Enantiomeren der Verbindungen der Formel I kommt man entweder durch Racematspaltung (über Salzbildung mit optisch aktiven Säuren oder Basen), oder indem man in die Synthese optisch aktive Ausgangsstoffe einsetzt oder indem man erzymatisch hydrolysiert.

Bevorzugt im Sinne der Erfindung sind außer den in den Beispielen genannten Verbindungen die folgenden:
1. ((7-Carbamimidoyl-naphthalin-2-yloxy)-{4-[1-(1-imino-ethyl)-piperidin-4-yloxy]-phenyl}-methyl)-phosphonsäurediallylester
2. {(7-Carbamimidoyl-naphthalin-2-yloxy)-[4-(1-carbamimidoyl-piperidin-4-yloxy)-phenyl]-methyl}-phosphonsäurediethylester
3. ((7-Carbamimidoyl-naphthalin-2-yloxy)-{4-[1-(1-imino-ethyl)-pyrrolidin-3-yloxy]-phenyl}-methyl)-phosphonsäurediethylester
4. {(7-Carbamimidoyl-naphthalin-2-yloxy)-[4-(1-carbamimidoyl-pyrrolidin-3-yloxy)-phenyl]-methyl}-phosphonsäurediethylester
5. ((7-Carbamimidoyl-naphthalin-2-yloxy)-{4-[1-(1-imino-ethyl)-piperidin-4-yloxy]-phenyl}-methyl)-phosphonsäuremonoethylester
6. {(7-Carbamimidoyl-naphthalin-2-yloxy)-[4-(1-carbamimidoyl-piperidin-4-yloxy)-phenyl]-methyl}-phosphonsäuremonoethylester
7. ((7-Carbamimidoyl-naphthalin-2-yloxy)-{4-[1-(1-imino-ethyl)-pyrrolidin-3-yloxy]-phenyl}-methyl)-phosphonsäuremonoethylester
8. {(7-Carbamimidoyl-naphthalin-2-yloxy)-[4-(1-carbamimidoyl-pyrrolidin-3-yloxy)-phenyl]-methyl}-phosphonsäuremonoethylester
9. ((7-Carbamimidoyl-naphthalin-2-yloxy)-{4-[1-(1-imino-propyl)-piperidin-4-yloxy]-phenyl}-methyl)-phosphonsäuremonoethylester
10. ((7-Carbamimidoyl-naphthalin-2-yloxy)-{4-[1-(1-imino-ethyl)-piperidin-4-yloxy]-phenyl}-methyl)-phosphonsäure
11. {(7-Carbamimidoyl-naphthalin-2-yloxy)-[4-(1-carbamimidoyl-piperidin-4-yloxy)-phenyl]-methyl}-phosphonsäure
12. ((7-Carbamimidoyl-naphthalin-2-yloxy)-{4-[1-(1-imino-ethyl)-pyrrolidin-3-yloxy]-phenyl}-methyl)-phosphonsäure
13. {(7-Carbamimidoyl-naphthalin-2-yloxy)-[4-(1-carbamimidoyl-pyrrolidin-3-yloxy)-phenyl]-methyl}-phosphonsäure
14. ((7-Carbamimidoyl-naphthalin-2-yloxy)-{4-[1-(1-imino-ethyl)-piperidin-4-yloxy]-phenyl}-methyl)-phosphonsäuredimethylester
15. {(7-Carbamimidoyl-naphthalin-2-yloxy)-[4-(1-carbamimidoyl-piperidin-4-yloxy)-phenyl]-methyl}-phosphonsäuredimethylester
16. ((7-Carbamimidoyl-naphthalin-2-yloxy)-{4-[1-(1-imino-ethyl)-pyrrolidin-3-yloxy]-phenyl}-methyl)-phosphonsäuredimethylester
17. {(7-Carbamimidoyl-naphthalin-2-yloxy)-[4-(1-carbamimidoyl-pyrrolidin-3-yloxy)-phenyl]-methyl}-phosphonsäuredimethylester
18. ((7-Carbamimidoyl-naphthalin-2-yloxy)-{4-[1-(1-imino-ethyl)-piperidin-4-yloxy]-phenyl}-methyl)-phosphonsäuremonomethylester
19. {(7-Carbamimidoyl-naphthalin-2-yloxy)-[4-(1-carbamimidoyl-piperidin-4-yloxy)-phenyl]-methyl}-phosphonsäuremonomethylester
20. ((7-Carbamimidoyl-naphthalin-2-yloxy)-{4-[1-(1-imino-ethyl)-pyrrolidin-3-yloxy]-phenyl}-methyl)-phosphonsäuremonomethylester
21. {(7-Carbamimidoyl-naphthalin-2-yloxy)-[4-(1-carbamimidoyl-pyrrolidin-3-yloxy)-phenyl]-methyl}-phosphonsäuremonomethylester
22. ((7-Carbamimidoyl-naphthalin-2-yloxy)-{4-[1-(1-imino-propyl)-piperidin-4-yloxy]-phenyl}-methyl)-phosphonsäuremonomethylester
23. ((7-Carbamimidoyl-naphthalin-2-yloxy)-{4-[1-(1-imino-ethyl)-piperidin-4-yloxy]-phenyl}-methyl)-phosphonsäuredipropylester
24. {(7-Carbamimidoyl-naphthalin-2-yloxy)-[4-(1-carbamimidoyl-piperidin-4-yloxy)-phenyl]-methyl}-phosphonsäuredipropylester
25. ((7-Carbamimidoyl-naphthalin-2-yloxy)-{4-[1-(1-imino-ethyl)-pyrrolidin-3-yloxy]-phenyl}-methyl)-phosphonsäuredipropylester
26. {(7-Carbamimidoyl-naphthalin-2-yloxy)-[4-(1-carbamimidoyl-pyrrolidin-3-yloxy)-phenyl]-methyl}-phosphonsäuredipropylester
27. ((7-Carbamimidoyl-naphthalin-2-yloxy)-{4-[1-(1-imino-ethyl)-piperidin-4-yloxy]-phenyl}-methyl)-phosphonsäuredibenzylester
28. {(7-Carbamimidoyl-naphthalin-2-yloxy)-[4-(1-carbamimidoyl-piperidin-4-yloxy)-phenyl]-methyl}-phosphonsäuredibenzylester
29. ((7-Carbamimidoyl-naphthalin-2-yloxy)-{4-[1-(1-imino-ethyl)-pyrrolidin-3-yloxy]-phenyl}-methyl)-phosphonsäuredibenzylester
30. {(7-Carbamimidoyl-naphthalin-2-yloxy)-[4-(1-carbamimidoyl-pyrrolidin-3-yloxy)-phenyl]-methyl}-phosphonsäuredibenzylester

### Beispiel 1:

### ((7-Carbamimidoyl-naphthalin-2-yloxy)-{4-[1-(1-imino-ethyl)-piperidin-4-yloxy]-phenyl}-methyl)-phosphonsäurediethylester-dihydrochlorid

### 1. 4-(4-Formyl-phenoxy)-piperidin-1-carbonsäure-tert-butylester

Eine Lösung von 4.9 g (0.040 mol) 4-Hydroxybenzaldehyd, 10.0 g (0.050 mol) 4-Hydroxy-piperidin-1-carbonsäure-tert-butylester (analog K. L. Bhat, D. M. Flanagan, M. M. Jouillé, *Synth. Commun*. **1985**, *15*, 587-598) und 13.1 g (0.050 mol) Triphenylphosphin in 200 ml Tetrahydrofuran wird bei 5°C mit 7.9 ml (0.050 mol) Azodicarbonsäurediethylester versetzt und 24 h bei Raumtemperatur gerührt. Nach Einengen wird der Rückstand zur Reinigung an einer Kieselgelsäule chromatographiert (Laufmittel: Isohexan/Ethylacetat 8:2, 7:3). Nach Einengen der entsprechenden Säulenfraktionen erhält man 18.4 g (85%) der Titelverbindung als gelblichen Feststoff. Schmp. 63-64°C; EI-MS: 305 (M⁺).

### 2. 4-{4-[(Diethoxy-phosphoryl)-hydroxy-methyl]-phenoxy}-piperidin-1-carbonsäure-tert-butylester

Eine Mischung von 0.99 g (0.0032 mol) 4-(4-Formyl-phenoxy)-piperidin-1-carbonsäure-tert-butylester, 0.46 ml (0.0036 mol) Diethylphosphit und 0.50 ml Triethylamin (0.0036 mol) wird 4 h auf 60°C erhitzt. Nach Einengen wird der Rückstand zur Reinigung an einer Kieselgelsäule chromatographiert (Laufmittel: Ethylacetat/Methanol 19:1). Nach Einengen der entsprechenden Säulenfraktionen erhält man 1.40 g (97%) der Titelverbindung als viskoses, farbloses Öl. ³¹P-NMR: δ = 23.3 ppm; EI-MS (silyliert): 515 (M⁺- H + (CH₃)₃Si).

### 3. 4-{4-[(7-Cyano-naphthalin-2-yloxy)-(diethoxy-phosphoryl)-methyl]-phenoxy}-piperidin-1-carbonsäure-tert-butylester

Eine Lösung von 1.50 g (0.0088 mol) 7-Hydroxy-naphthalin-2-carbonitril, 3.93 g (0.0088 mol) 4-{4-[(Diethoxy-phosphoryl)-hydroxy-methyl]-phenoxy}-piperidin-1-carbonsäure-tert-butylester und 3.73 g (0.0142 mol) Triphenylphosphin in 150 ml Tetrahydrofuran wird bei 5°C mit 2.23 ml (0.0142 mol) Azodicarbonsäure-diethylester versetzt und 96 h bei Raumtemperatur gerührt. Nach Einengen wird der Rückstand zur Reinigung an einer Kieselgelsäule chromatographiert (Laufmittel: Ethylacetat/Methanol 19:1). Nach Einengen der entsprechenden Säulenfraktionen erhält man 2.47 g (47%) der Titelverbindung als viskoses, gelbes Öl. ³¹P-NMR: δ = 19.0 ppm.

### 4. 7-{[4-(1-tert-Butoxycarbonyl-piperidin-4-yloxy)-phenyl]-(diethoxy-phosphoryl)}-methyl-oxy-naphthalin-2-carbothioamid

Eine Lösung von 1.61 g (0.0027 mmol) 4-{4-[(7-Cyano-naphthalin-2-yloxy)-(diethoxy-phosphoryl)-methyl]-phenoxy}-piperidin-1-carbonsäure-tert-butylester und 0.75 ml (0.0054 mmol) Triethylamin in 15 ml Pyridin wird mit Schwefelwasserstoff gesättigt. Man rührt 2 d bei Raumtemperatur, entfernt alle flüchtigen Komponenten im Vakuum und erhält 1.67 g (98%) der Titelverbindung als orangefarbenen Feststoff. ³¹P-NMR: δ = 19.5 ppm; (-)-FAB-MS: 627 (M-H⁺).

### 5. 7-{[4-(1-tert-Butoxycarbonyl-piperidin-4-yloxy)-phenyl]-(diethoxy-phosphoryl)}-methyl-oxy-naphthalin-2-carbimidothionsäuremethylester-hydroiodid

Eine Lösung von 1.56 g (0.0025 mol) 7-{[4-(1-tert-Butoxycarbonyl-piperidin-4-yloxy)-phenyl]-(diethoxy-phosphoryl)}-methyl-oxy-naphthalin-2-carbothioamid in 15 ml Aceton wird mit 0.77 ml (0.012 mol) Methyliodid versetzt und unter Lichtausschluß 24 h bei Raumtemperatur gerührt. Man entfernt alle flüchtigen Komponenten im Vakuum und erhält 2.38 g der Titelverbindung als gelben Feststoff (³¹P-NMR: δ = 19.2 ppm). Das Rohprodukt wird ohne Aufreinigung weiter umgesetzt.

### 6. 7-{[4-(Piperidin-4-yloxy)-phenyl]-(diethoxy-phosphoryl)}-methyloxy-naphthalin-2-carbamidin-dihydrochlorid

Eine Mischung von 2.27 g (0.003 mmol) 7-{[4-(1-tert-Butoxycarbonyl-piperidin-4-yloxy)-phenyl]-(diethoxy-phosphoryl)}-methyl-oxy-naphthalin-2-carbimidothion-säuremethylester-hydroiodid und 0.68 g (0.0089) Ammoniumacetat in 20 ml Methanol wird 24 h unter Rückfluß erhitzt. Nach Abkühlen des Reaktionsgemisches werden unter Eiskühlung innerhalb von 4 h 20 ml einer mit Chlorwasserstoff gesättigten Diethylether-Lösung zugetropft. Nach Entfernen des Lösungsmittels im Vakuum wird der Rückstand wird in 25 ml Wasser gelöst, mit 2 N HCl auf pH 3 eingestellt und mittels präparativer HPLC (RP-18-Säule, 15-25µm) chromatographiert (Laufmittel: H₂O/CH₃OH 55:45, pH 3). Nach Einengen der entsprechenden Säulenfraktionen und Trocknen i. Vak. (10⁻² Torr) wird der Rückstand in 5 ml Wasser / tert.-Butanol Gemisch (1:1) gelöst und lyophilisiert. Man erhält man 0.66 g (0.0011 mol) der Titelverbindung als schwach rosafarbenen Feststoff mit Schmp. 278°C. ³¹P-NMR: δ = 19.0 ppm; (+)-FAB-MS: 512 (M+H⁺).

### 7. ((7-Carbamimidoyl-naphthalin-2-yloxy)-{4-[1-(1-imino-ethyl)-piperidin-4-yloxy]-phenyl}-methyl)-phosphonsäurediethylester-dihydrochlorid

Zu 42 mg Acetimidsäureethylester-hydrochlorid (0.34 mmol) und 100 mg 7-{[4-(Piperidin-4-yloxy)-phenyl]-(diethoxy-phosphoryl)}-methyloxy-naphthalin-2-carbamidin-dihydrochlorid (0.17 mmol) in 10 ml Ethanol werden unter Stickstoff bei 5°C 0.24 ml (1.71 mol) Triethylamin getropft. Man rührt 2 Tage bei Raumtemperatur, dampft ein, löst den Rückstand in 10 ml Wasser, stellt mit 2 N HCl auf pH 3 ein und filtriert. Das Filtrat wird mittels präparativer HPLC (RP-18-Säule, 15-25µm) chromatographiert (Laufmittel: H₂O, pH 3; H₂O/CH₃CN 65:35, pH 3). Nach Einengen der entsprechenden Säulenfiaktionen und Trocknen im Vakuum (10⁻² Torr) erhält man 70 mg (0.11 mmol; 65 %) der Titelverbindung als helles, erstarrtes Öl. (+)-FAB-MS: 553 (MH⁺).

## Patentansprüche

1. Verbindungen der Formel I 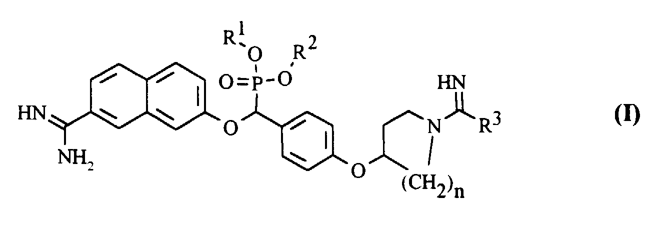 in der
R¹, R² gleich oder verschieden sind und ein Wasserstoffatom, eine Alkylgruppe, eine Cycloalkylgruppe, eine Hydroxyalkylgruppe, eine Alkenylgruppe, eine Alkinylgruppe oder eine Aralkylgruppe bedeuten oder R¹ und R² zusammen einen Alkrylenrest bedeuten, der mit den gebundenenen Sauerstoffatomen und dem die Sauerstoffatome tragenden Phosphoratom einen gesättigten 5- bis 8-gliedrigen Ring bildet;
R³ eine gegebenenfalls substituierte Aminogruppe, eine Alkylgruppe, einen Cycloalkylrest oder einen gegebenenfalls substituierten Alylrest bedeutet;
n eine ganze Zahl zwischen 1 und 4 bedeutet,
sowie Hydrate, Solvate und physiologisch verträgliche Salze davon, und deren optisch aktiven Formen.

2. Verbindungen der Formel I gemäß Anspruch 1, dadurch gekennzeichnet, daß
R¹, R² gleich oder verschieden sind und ein Wasserstoffatom, eine Methylgruppe, eine Ethylgruppe, eine Propylgruppe, eine Allylgruppe, eine Propargylgruppe oder eine Benzylgruppe bedeuten, oder R¹ und R² zusammen eine Ethylengruppe oder eine Propylengruppe bedeuten;
R³ eine Aminogruppe, eine N-Methyl-aminogruppe, eine N-Bensyl-aminogruppe, eine N-Allyl-aminogruppe, eine N,N-Dimethylaminogruppe, eine Methylgruppe, eine Ethylgruppe, eine Cyclopropylgruppe oder einen 4-Methoxyphenylrest bedeutet und
n 1 oder 2 sein kann.

3. Arzneimittel, enthaltend mindestens eine Verbindung der Formel I gemäß Anspruch 1 oder 2 neben üblichen Träger- und Hilfsstoffen.

4. Verwendung von Verbindungen der Formel I gemäß Anspruch 1 oder 2 zur Herstellung von Arzneimitteln zur Behandlung oder Prophylaxe von Thrombosen, Apoplexie, Herzinfarkt, Entzündungen und Artereosklerose.

5. Verwendung von Verbindungen der Formel I gemäß Anspruch 1 oder 2 zur Herstellung von Arzneimitteln mit Faktor Xa-inhibierenden Eigenschaften.
